# DEMANDE DE BREVET EUROPEEN

(11) **EP 0 653 428 A1**
(43) Date de publication de la demande: **17.05.1995**
(21) Numéro de dépôt: 94402585.7
(22) Date de dépôt: 16.11.1994
(51) Int. Cl.: C07D 498/10, G03C 1/685

(54) **Spirooxazines et leur utilisation dans des lentilles opthalmiques**

(30) Priorité: 17.11.1993 FR 9313731
(71) Demandeur: ESSILOR INTERNATIONAL (COMPAGNIE GENERALE D'OPTIQUE), F-94227 Charenton cédex (FR)
(72) Inventeur: Guglielmetti, Robert, Boulevard des Alisiers F-13009 Marseille (FR); Samat, André, F-13013 Marseille (FR); Laregnie, Pierre, F-13008 Paris (FR)
(74) Mandataire: Casalonga, Axel

(57) **Abrégé**

L'invention est relative à des composés photochromiques de formule générale : 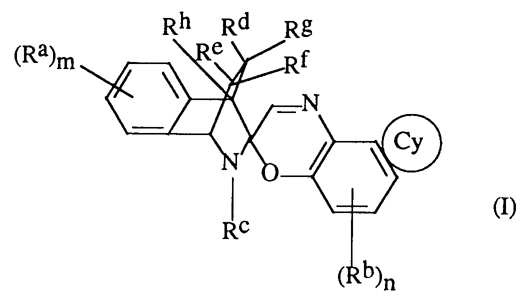
dans laquelle :
R^{a} et R^{b} désignent hydrogène; alkyle; OR, SR, COR ou COOR où R désigne hydrogène, alkyle, aryle ou hétéroaryle; un groupe NR₁ R₂ où R₁ R₂ désigne hydrogène, alkyle ou cycloalkyle en C₄-C₇, aryle, forment avec l'azote un hétérocycle en C₄-C₇; NO₂, CN, SCN ou un halogène; un groupe SO₃R' où R' désigne hydrogène ou un métal alcalin; un mono ou polyhaloalkyle; m désigne un entier de 1 à 4 et n vaut 1 ou 2;
C_{y} désigne un cycle aromatique ou un hétérocycle aromatique ou non;
R^{c} désigne un groupe alkyle, allyle, phényle, arylalkyle mono ou disubstitué par alkyle, alcoxy ou NO₂; un alicyclique, un hydrocarbure aliphatique comportant un ou plusieurs hétéroatomes;
R^{d}, R^{e}, R^{f}, R^{g} désignent hydrogène, alkyle, alcoxy ou thioalkyle ou forment deux à deux un cycloalkyle ayant 4 à 7 chaînons pouvant comporter des hétéroatomes éventuellement condensés par un noyau aromatique; R^{h} désigne hydrogène ou forme un cycloalkyle à 5 ou 6 chaînons avec R^{d}.
et leur utilisation dans l'optique ophtalmique.

## Description

L'invention a pour objet de nouveaux composés photochromiques, plus particulièrement des composés photochromiques comportant dans leur formule chimique un noyau de la famille des spirooxazines, leur utilisation dans le domaine de l'optique ophtalmique, en particulier dans et/ou sur les lentilles ophtalmiques.

Le photochromisme est un phénomène connu depuis de nombreuses années. On dit qu'un composé est photochromique lorsque ce composé, irradié par un faisceau lumineux, dont certaines longueurs d'onde se situent dans le domaine de l'ultraviolet, change de couleur et revient à sa couleur originelle dès que l'irradiation cesse.

Les applications de ce phénomène sont multiples, mais une des applications connues p lus particulièrement intéressante concerne le domaine de l'optique ophtalmique.

De tels composés sont utilisables dans la fabrication de lentilles ou verres pour lunettes en vue de filtrer les radiations lumineuses en fonction de leur intensité.

L'incorporation des composés photochromiques dans un matériau organique constituant une lentille ophtalmique, permet d'obtenir un verre dont le poids est considérablement réduit par rapport aux lentilles classiques en verre minéral qui comportent des halogénures d'argent à titre d'agent photochrome. Leur incorporation dans des matériaux organiques a toujours posé des difficultés techniques.

Toutefois, tout composé à propriété photochromique n'est pas forcément utilisable dans le domaine de l'optique ophtalmique. En effet, le composé photochromique doit répondre à un certain nombre de critères, dont entre autres :
- une forte colorabilité qui est la mesure de la capacité pour un composé photochromique de présenter une couleur intense après isomérisation;
- une coloration après absorption de la lumière rendant apte le composé photochromique, seul ou en combinaison avec d'autres composés photochromiques à être utilisé dans des verres ou lentilles ophtalmiques;
- une absence de coloration ou très faible coloration sous la forme initiale;
- une cinétique rapide de coloration ou de décoloration;
- un photochromisme se manifestant dans une plage de température la plus large possible, et en particulier de préférence entre 0 et 40°C.

Les composés photochromiques organiques connus et utilisés actuellement présentent généralement un photochromisme décroissant lorsque la température augmente, de sorte que le photochromisme est particulièrement marqué à des températures proches de 0°C, alors qu'il est beaucoup plus faible, voire inexistant, à des températures de l'ordre de 40°C qui sont des températures que peuvent atteindre les verres lors notamment de l'exposition au soleil.

Un autre problème rencontré pour les composés photochromiques de l'état de la technique est leur durée de vie. On constate en effet pour certains produits de l'état de la technique, une durée de vie relativement réduite. En effet, après un certain nombre de cycles de coloration et de décoloration, le composé photochromique se bloque généralement dans une forme ouverte et colorée et ne présente plus les propriétés photochromes réversibles.

Les propriétés photochromiques des spirooxazines ont été décrites par R.E.FOX dans le document Final Report of Contact AF 41, A.D. 440226 1961. 657.

Des composés du type spiro(indoline-naphtooxazine) ont été synthétisés et décrits notamment dans l'article de N.Y.C. CHU "Photochromisme: Molecules and Systems" Ed. H. Dürr, H. Bovas Laurent, Elsevier, Amsterdam 1990, ch. 24, ainsi que des composés du type spiro(indoline-quinazolinooxazine) ou spiro(indoline-benzothiazolooxazine) dans les brevets US 5139707 et US 5114621 (R. Guglielmetti, P. Tardieu) délivrés au nom de la Société ESSILOR.

La Société demanderesse a découvert une nouvelle famille de spirooxazines présentant des propriétés photochromiques particulièrement intéressantes. Parmi les composés appartenant à cette famille, un grand nombre de composés présentent en effet une forte colorabilité en particulier dans le domaine rouge particulièrement utile pour l'optique ophtalmique, ces composés pouvant être utilisés avec des composés photochromiques donnant une couleur bleue, en vue d'obtenir une coloration finale naturelle lors de l'exposition à la lumière. Ils présentent, en outre, sous leur forme colorée, un déplacement hypsochromique au niveau de l'absorption de la lumière, plus important et une colorabilité similaire ou renforcée par rapport aux composés spirooxazine connus dans l'état de la technique.

D'autres composés spirooxazines selon l'invention sous leur forme colorée, présentent une couleur résultant d'une bande d'absorption très large dans le visible. L'utilisation de ces composés particuliers dans l'optique ophtalmique, permet de limiter le nombre de composés photochromiques de nature différente à mettre en oeuvre pour l'obtention des teintes spécifiques aux verres photochromiques courants.

Les composés conformes à l'invention présentent par ailleurs une absence de coloration ou une très faible coloration à l'état initial et une cinétique rapide de coloration et décoloration dans un plage de température très large, comprise en particulier entre 0 et 40°C.

La demanderesse a également constaté que ces composés avaient une bonne durée de vie.

Toutes ces propriétés font que ces nouveaux composés photochromiques sont particulièrement intéressants dans leur utilisation dans l'optique ophtalmique et en particulier pour leur utilisation dans et/ou sur des lentilles ophtalmiques.

On appelle lentilles ophtalmiques au sens de l'invention, des verres de lunettes, en particulier des verres solaires, des lentilles de contact.

Un objet de l'invention est donc constitué par les nouveaux composés photochromiques.

Un autre objet de l'invention est constitué par leur utilisation dans l'optique ophtalmique.

L'invention a également pour objet des compositions destinées à être utilisées pour le revêtement de lentilles ophtalmiques ou leur incorporation dans ces lentilles.

D'autres objets de l'invention apparaîtront à la lecture de la description et des exemples qui suivent.

Le composé photochromique conforme à l'invention est essentiellement caractérisé par le fait qu'il répond à la formule générale : 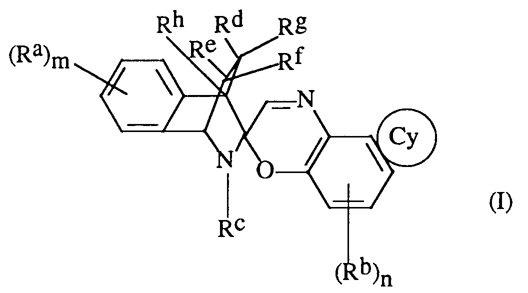
dans laquelle
R^{a} et R^{b}, indépendamment l'un de l'autre, désignent un atome d'hydrogène; un groupement alkyle; un groupement OR, SR, COR ou COOR ou SO₂R dans lesquels R désigne un atome d'hydrogène, un groupement alkyle, aryle ou hétéroaryle; un groupement amino NR₁R₂ dans lequel R₁ et R₂ désignent, indépendamment l'un de l'autre, un atome d'hydrogène, un groupement alkyle, un groupe cycloalkyle, un groupement aryle, R₁ et R₂ pouvant former avec l'atome d'azote un hétérocycle comportant 4 à 7 chaînons et pouvant contenir en plus un ou plusieurs hétéroatomes choisis parmi soufre, oxygène et azote; un atome d'halogène, un groupe monohaloalkyle en C₁-C₄; un groupe polyhaloalkyle en C₁-C₄; un groupe NO₂, CN, SCN; SO₃R' où R' désigne hydrogène ou un métal alcalin;
m désigne un entier de 1 à 4 et n vaut 1 ou 2 selon le nombre de substitutions sur le noyau;
Cy désigne un cycle aromatique, de préférence à 5 ou 6 chaînons pouvant être substitué par un ou plusieurs groupements ayant la signification de R^{a} ou un hétérocycle aromatique ou non comportant un ou plusieurs hétéroatomes choisis parmi l'azote, l'oxygène et le soufre ayant 4 à 7 chaînons.

R^{c} désigne un groupe alkyle; un groupe allyle, phényle, arylalkyle mono ou disubstitué par des substituants du type alkyle, alcoxy ou NO₂; un groupe alicyclique éventuellement substitué; un groupe hydrocarboné aliphatique comportant dans sa chaîne un ou plusieurs hétéroatomes tels que oxygène, soufre ou azote.

R^{d}, R^{e}, R^{f}, R^{g} désignent, indépendamment l'un de l'autre, un atome d'hydrogène, un groupement alkyle, un groupe alcoxy ou un groupe thioalkyle; deux de ces radicaux peuvent former un groupement cycloalkyle ou cycloalcényle ayant 4 à 7 chaînons, pouvant comporter un ou plusieurs hétéroatomes choisis parmi l'azote, le soufre, l'oxygène et pouvant être condensés avec un noyau aromatique à 5 ou 6 chaînons pouvant être substitué par un ou plusieurs radicaux ayant la signification de R^{a} et R^{b}.

R^{h} désigne hydrogène ou forme avec R^{d} un cycloalkyle à 5 ou 6 chaînons.

De préférence, l'un au moins des radicaux R^{d}, R^{e} n'est pas un atome d'hydrogène.

Dans la formule précitée, les groupements alcoxy et alkyle comportent de préférence 1 à 6 atomes de carbone tels que méthoxy, éthoxy, méthyle, éthyle, propyle; les groupements cycloalkyle sont de préférence cyclohexyle ou cyclopentyle; les groupes cycloalcényle sont de préférence cyclohexényle ou cyclopentényle, le groupement aryle est de préférence phényle; le groupement arylalkyle est de préférence benzyle; le groupe polyhaloalkyle est par exemple CF₃; l'halogène est notamment fluor, brome ou chlore.

Les noyaux aromatiques à 5 ou 6 chaînons sont notamment phényle, naphtyle.

Les noyaux hétérocycles à 4 à 7 chaînons sont notamment les cycles pyridinique, pyrimidique, indolique, imidazolique ou thiazolique éventuellement substitués par un groupe alkyle; un groupement phényle ou amino; un groupement carbonyle ou un halogène. Le groupement hydrocarboné aliphatique comporte de préférence une fonction acide, ester ou alcool.

Les composés préférentiels de l'invention sont ceux de formule (I) pour lesquels les radicaux R^{a} et R^{b} désignent H ou NO₂; C_{Y} désigne un cycle aromatique à 6 chaînons éventuellement substitué par un alcoxy; R^{c} est un groupe alkyle, de préférence méthyle; R^{d}, R^{e}, R^{f}, R^{g} désignent un groupe alcoxy, de préférence éthoxy ou deux d'entre eux forment un cycloalcényle, de préférence cyclopentényle.

Les composés de l'invention particulièrement préférés sont ceux répondant à l'une des formules suivantes :

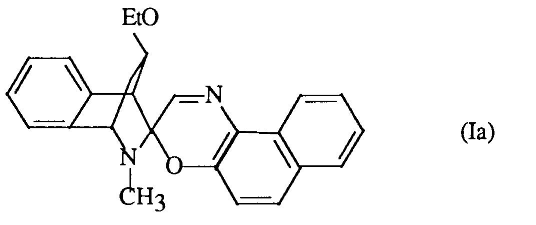

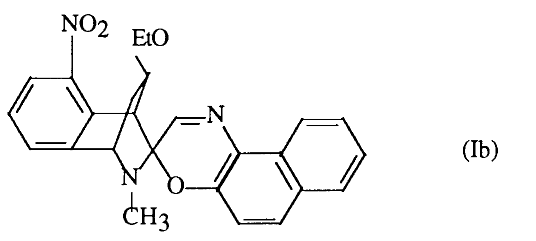

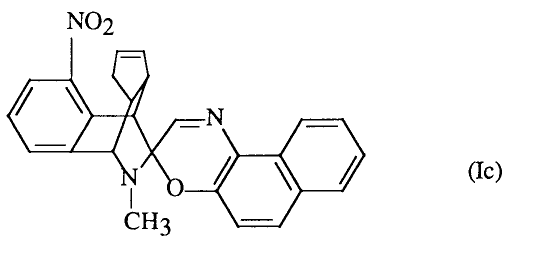

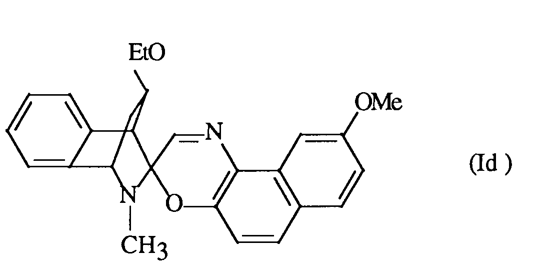

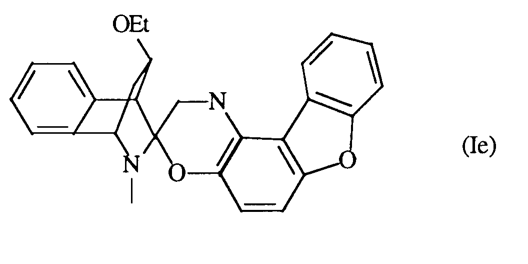

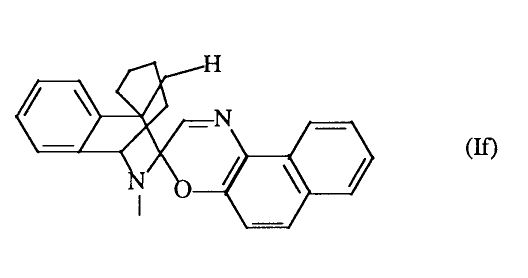

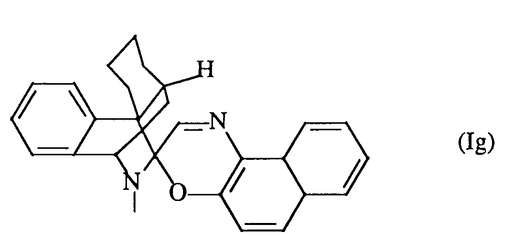

Les composés conformes à l'invention peuvent être préparés selon le schéma réactionnel suivant :

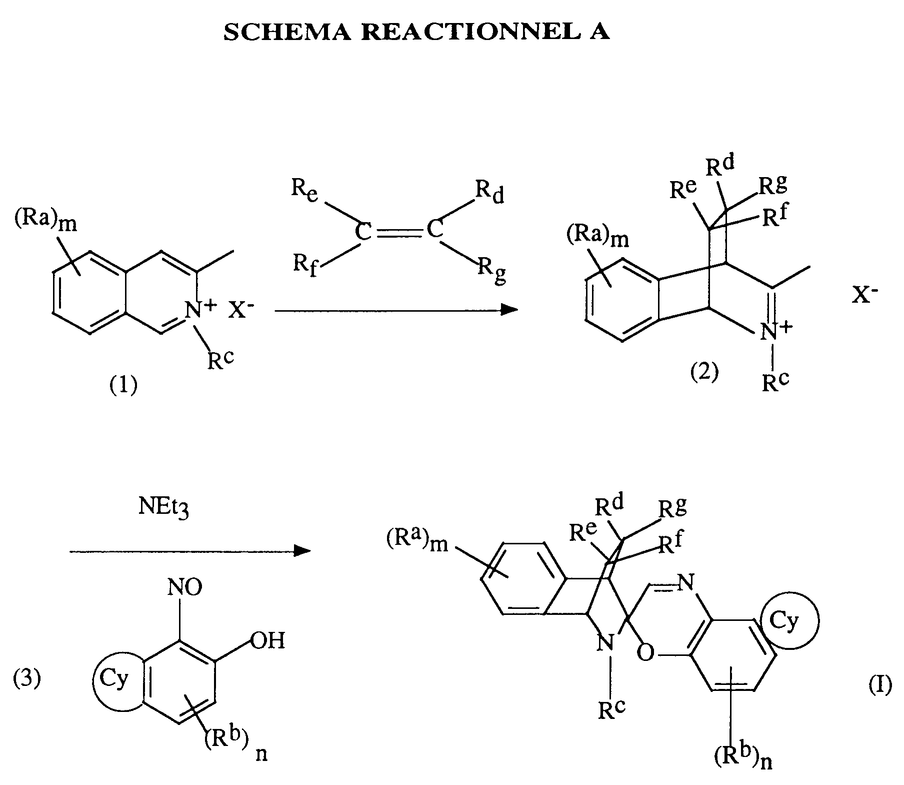

Dans les formules (1), (2) et (3), les radicaux R^{a}, R^{b}, R^{c}, R^{d}, R^{e}, R^{f}, R^{g}, C_{y} ont les mêmes significations indiquées ci-dessus et X⁻ désigne un anion de préférence un halogénure.

Les composés photochromiques de l'invention peuvent être utilisés pour réaliser des lentilles ophtalmiques photochromiques.

Les composés conformes à l'invention peuvent être introduits dans une composition destinée à être appliquée sur ou être introduite dans un matériau polymère organique transparent pour obtenir un article transparent photochromique. ns peuvent également être introduits dans des compositions solides telles que films plastique, plaques et lentilles pour réaliser des matériaux utilisables notamment comme lentilles ophtalmiques, lunettes de soleil, viseurs, optique de caméra et filtres.

Les compositions liquides qui constituent un objet de l'invention sont essentiellement caractérisées par le fait qu'elles contiennent sous forme dissoute ou dispersée les composés conformes à l'invention dans un milieu à base de solvants appropriés pour être appliqués ou introduits dans un matériau polymère transparent.

Des solvants plus particulièrement utilisables sont des solvants organiques choisis parmi le benzène, le toluène, le chloroforme, l'acétate d'éthyle, la méthyléthylcétone, I'acétone, l'alcool éthylique, l'alcool méthylique, l'acétonitrile, le tétrahydrofurane, le dioxane, l'éther méthylique d'éthylèneglycol, le diméthylformamide, le diméthylsulfoxyde, le méthylcellosolve, la morpholine, et l'éthylèneglycol.

Lorsque les composés conformes à l'invention sont dispersés, le milieu peut également contenir de l'eau.

Selon une autre forme de réalisation, les composés conformes à l'invention peuvent être introduits et de préférence dissous dans des solutions incolores ou transparentes préparées à partir de polymères, de copolymères ou de mélanges de polymères transparents dans un solvant organique approprié.

Les exemples de telles solutions sont entre autres des solutions de nitrocellulose dans l'acétonitrile, de polyvinylacétate dans l'acétone, de chlorure de polyvinyle dans la méthyléthylcétone, d'acétylcellulose dans le diméthylformamide, de polyvinylpyrrolidone dans de l'acétonitrile, de polystyrène dans le benzène, d'éthylcellulose dans du chlorure de méthylène.

Ces compositions peuvent être appliquées sur des supports transparents tels qu'en téréphtalate de polyéthylèneglycol, de papier borylé, de triacétate de cellulose et séchées pour obtenir un matériau photochromique qui peut se colorer en présence d'une radiation ultraviolette, et qui retourne à l'état non coloré et transparent en l'absence de la source de radiation.

Les composés photochromiques de la présente invention ou les compositions les contenant définies ci-dessus peuvent être appliqués ou incorporés dans un matériau organique polymérisé transparent solide approprié pour des éléments ophtamiques tels que des lentilles ophtamiques ou des matériaux utiles pour être utilisés dans des lunettes de soleil, des viseurs, des optiques de caméras et des filtres.

A titre de matériaux solides transparents, qui peuvent être utilisés pour réaliser des lentilles ophtalmiques conforme à l'invention, on peut citer les polymères de polyol(allylcarbonate), des polyacrylates, des poly(alkylacrylate) tels que des polyméthylméthacrylates, l'acétate de cellulose, le triacétate de cellulose, le propionate acétate de cellulose, le butyrate acétate de cellulose, le poly(vinylacétate), le poly(vinylalcool), les polyuréthanes, les polycarbonates, les polyéthylènetéréphtalates, les polystyrènes, les (polystyrèneméthylméthacrylate), les copolymères de styrène et d'acrylonitrile, les polyvinylbutyrates.

Les copolymères transparents ou des mélanges de polymères transparents sont également appropriés pour réaliser de tels matériaux.

On peut citer à ce sujet les matériaux préparés à partir de polycarbonates tels que le poly(4,4'-dioxyphényl-2,2 propanecarbonate), le polyméthylméthacrylate, les polyol(allylcarbonate) tels qu'en particulier le diéthylèneglycol bis(allylcarbonate) et ses copolymères tels que par exemple avec l'acétate de vinyle. On peut citer en particulier les copolymères de diéthylèneglycol bis(allylcarbonate) et d'acétate de vinyle (80-90/10-20) et encore le copolymère de diéthylèneglycol bis(allylcarbonate) avec l'acétate de vinyle, l'acétate de cellulose et le propionate de cellulose, le butyrate de cellulose (80-85/15-20).

Les polyols(allylcarbonate) sont préparés en utilisant les allylcarbonates de polyols liquides aliphatiques ou aromatiques, linéaires ou ramifiés tels que les glycols aliphatiques de bisallylcarbonate ou les alkylène bis(allylcarbonate). Parmi les polyols (allylcarbonate) qui peuvent être utilisés pour préparer les matériaux transparents solides utilisables conformément à l'iinvention, on peut citer l'éthylèneglycol bis(allylcarbonate), le diéthylèneglycol bis(2-méthallylcarbonate), le diéthylèneglycol bis(allylcarbonate), l'éthylèneglycol bis(2-chloroallylcarbonate), le triéthylèneglycol bis(allylcarbonate), le 1,3-propanediol bis(allylcarbonate), le propylèneglycol bis(2-éthylallylcarbonate), le 1,3-butanediol bis(allylcarbonate), le 1,4-butanediol bis(2-bromoallylcarbonate), le dipropylèneglycol bis(allylcarbonate), le triméthylèneglycol bis(2-éthylallylcarbonate), le pentaméthylèneglycol bis(allylcarbonate), l'isopropylène bisphénol bis (allylcarbonate). Le produit le plus important est constitué par le diéthylèneglycol bis(allylcarbonate) encore connu sous la dénomination CR39.

La quantité de composés photochromiques à utiliser conformément à l'invention, soit dans la composition, soit au moment de son introduction dans le support solide, n'est pas critique et dépend généralement de l'intensité de la couleur que la composition peut conférer au matériau après exposition aux radiations. D'une manière générale, plus on ajoute de composés photochromiques, plus la coloration sous irradiation sera importante.

Conformément à l'invention, on utilise une quantité suffisante pour conférer au matériau traité la propriété de changer de couleur au moment de l'exposition à la radiation. Cette quantité de composés photochromiques est généralement comprise entre 0,001 et 20% en poids et de préférence entre 0,05 et 10% en poids par rapport au poids total du matériau optique ou de la composition.

Les composés photochromiques conformes à l'invention peuvent également être introduits dans un support temporaire (tel qu'un vernis formant un revêtement sur un substrat) de transfert et être transférés ensuite thermiquement dans le substrat comme décrit en particulier dans le brevet US-4 286 957 ou US-4 880 667.

Ces composés peuvent être utilisés avec d'autres composés photochromiques, tels que des composés photochromiques donnant lieu à des colorations différentes telles que bleu, vert, connus dans l'état de la technique. C'est ainsi qu'on peut utiliser des spiro(indoline-oxazines) bien connus dans l'état de la technique.

Une fois appliqués sur des matériaux ophtalmiques ou introduits dans de tels matériaux, on constate après exposition aux irradiations UV, l'apparition d'une coloration et le retour à la couleur ou à la transparence originelle lorsqu'on interrompt l'exposition aux radiations UV.

Les composés conforme à l'invention présentent l'intérêt de permettre ce changement de coloration un grand nombre de fois et ceci à des températures très variables comprises entre 0 et 40°C.

Les exemples suivants sont destinés à illustrer l'invention sans pour autant présenter un caractère limitatif.

### Exemple 1

**Synthèse de la 8-éthoxy-2-méthylspiro[syn-5,6-benzo-2-azabicyclo-(2,2,2)octane-3,3'-[3H]napht-[2,1,b][1,4]oxazine] :**

### 1ère étape :

Synthèse de l'iodure de 2,3-diméthyl-8-éthoxy-(syn-5,6-benzo-2-azabicyclo-(2,2,2)octane) (1) obtenu selon la méthode de Bradscher (Bradscher C.K.; F. Day; Heterocycl. Chem. 11.23 (1974),

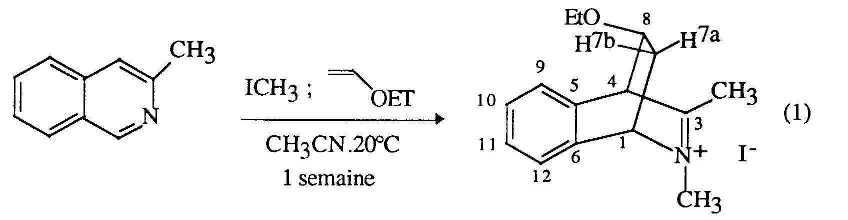

A une solution de 3-méthylisoquinoline (0,7 g; 4,9 mmoles) dans 35 ml d'acétonitrile, on ajoute 0,5 ml d'iodure de méthyle (7,3 mmol), 2,3 ml d'éthylvinyléther (24 mmoles) et 35 mg de 4-méthoxy phénol (0,28 mmole).

Le mélange réactionnel est laissé 7 jours sous agitation, à température ambiante, puis on obtient le produit désiré par précipitation (ajout d'éther éthylique).

### 2ème étape :

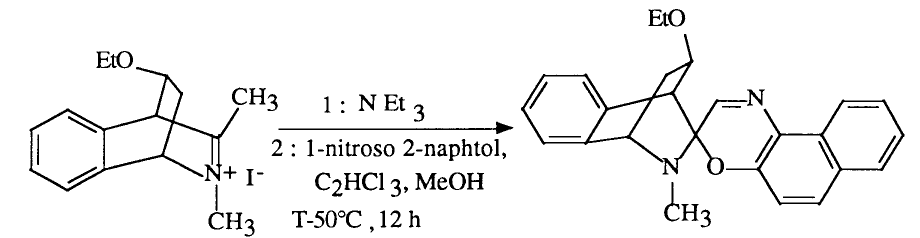

L'iodure de 2,3-diméthyl-8-éthoxy-(syn-5,6-benzo-2-azabicyclo-(2,2,2-octane) (0,1 g; 0,28mmole), est dissous dans 10 ml de trichloréthylène.

On ajoute alors un équivalent de triéthylamine (0,028 g; 0,28 mmole) puis quelques gouttes de méthanol (environ 1 ml) afin d'homogénéiser la solution.

Une solution de 1-nitroso 2-naphtol(0,048 g; 0,28 mmole) dans 5 ml de trichloréthylène, est additionnée goutte à goutte pendant 20 minutes à une température de 50°C. Le mélange réactionnel est laissé 12 heures, à 50°C, sous atmosphère d'azote.

L'évaporation du solvant donne une huile qui est chromatographiée sur gel de silice par chromatographie flash, en utilisant comme mélange éluant (95) Hexane/(5) Acétate d'éthyle.

La recristallisation dans le méthanol (T<50°C) permet d'obtenir la spironaphtoxazine attendue, et d'isoler un des diastéréoisomères : 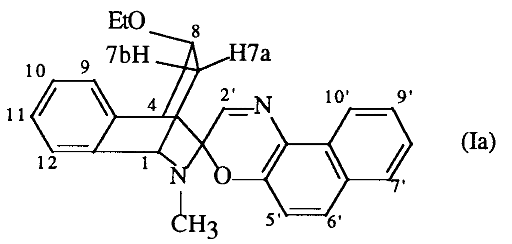
T_{F} =137-8°C

### Exemple 2

**Synthèse de la 8-éthoxy-2-méthyl-9-nitrospiro[syn-5,6-benzo-2-azabicyclo-(2,2,2)octane-3,3'-[3H]napht-[2,1,b][1,4]oxazine] :**

### 1ère étape :

Synthèse de l'iodure de 2,3-diméthyl-8-éthoxy-9-nitro(syn-5,6-benzo-2-azabicyclo-(2,2,2)octane) (4) :

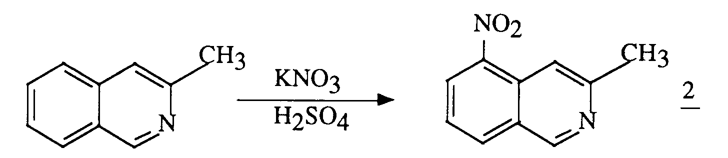

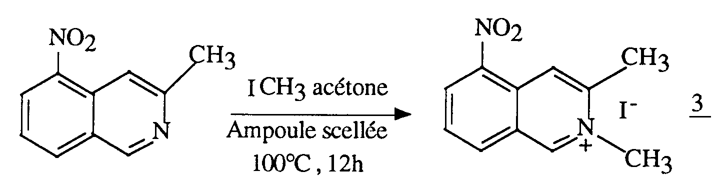

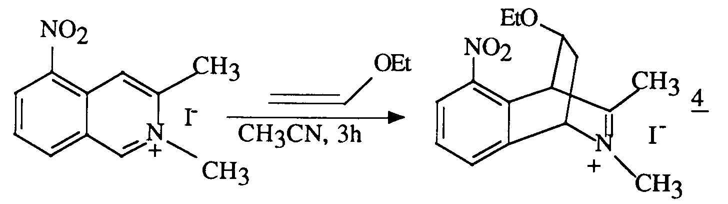

A une solution de 3-méthylisoquinoline (0,72 g; 5 mmoles) dissoute dans 4 ml d'acide sulfurique, placée dans un bain de glace, on additionne goutte à goutte une solution de nitrate de potassium (0,55 g; 5,4 mmoles) dissous dans 3 ml d'acide sulfurique, la température du milieu réactionnel ne devant excéder 4°C.

On verse alors dans une solution composée de 40 ml d'eau et 40 g de glace pilée, puis neutralise avec de l'ammoniaque, filtre et recristallise dans l'éthanol : on obtient la 3-méthyl-5-nitroisoquinoline 2.

La quaternisation de l'azote est effectuée en ampoule scellée : à 0,73 g (3,9 mmoles) de 3-méthyl-5-nitroisoquinoline dans 10 ml d'acétone, on ajoute 1,65 g (11,6 mmoles) d'iodure de méthyle.

On laisse une nuit à 100°C et récupère, après filtration, l'iodure de 2,3-diméthyl-5-nitro-isoquinolinium 3.

L'iodure de 2,3-diméthyl-5-nitroisoquinolinium (0,5g; 1,5 mmole) est dissous dans 10 ml d'acétonitrile; on ajoute alors 0,546 g (7,6 mmoles) d'éthylvinyl éther et 20 mg de 4-méthoxy phénol (0,16 mmole).

Le mélange réactionnel est laissé 3 heures à température ambiante, puis on recueille l'iodure azabicyclique 4 par précipitation dans l'éther éthylique et filtration.

### 2ème étape :

L'iodure de 2,3-diméthyl-8-éthoxy-9-nitro(syn-5,6-benzo-2-azabicyclo-(2,2,2)octane) (4) (0,1 g; 0,26 mmole) est dissous dans 10 ml de trichloréthylène. On ajoute alors un équivalent de triéthylamine (0,026 g; 0,26 mmole) puis quelques gouttes de méthanol (environ 1 ml) afin d'homogénéiser la solution.

Une solution de 1-nitroso 2-naphtol (0,045 g; 0,26 mmole) dans 10 ml de trichloréthylène est additionnée goutte à goutte pendant 20 minutes à une température de 50°C. Le mélange réactionnel est laissé 12 heures, à 50°C, sous atmosphère d'azote.

L'évaporation du solvant donne une huile qui est chromatographiée sur gel de silice par chromatographie flash, en utilisant comme mélange éluant (90) Hexane/(10) Acétate d'éthyle.

La recristallisation dans le méthanol (T<50°C) permet d'obtenir la spironaphtoxazine attendue (Ib).

8-éthoxy-2-méthyl-9-nitrospiro{syn-5,6-benzo-2-azabicyclo-(2,2,2)octane-3,3'-[3H]napht-[2,1,b][1,4]oxazine} : 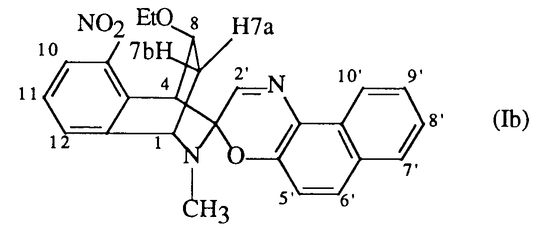
T_{f} = 139-143°C

### Exemple 3

**Synthèse de la 8-méthyl-15-nitrospiro[syn-10,11-benzo-8-azatricyclo-(5,2,2,0**^{**2,6**}**)undéc-3-ène-9,3'-[3H]napht-[2,1,b] [1,4]-oxazine]**

### 1ère étape :

Sythèse de l'iodure de 8,9-diméthyl-15-nitro[syn-10,11-benzo-8-azatricyclo-(5,2,2,0^{2,6})undéc-3-éne] (5) :

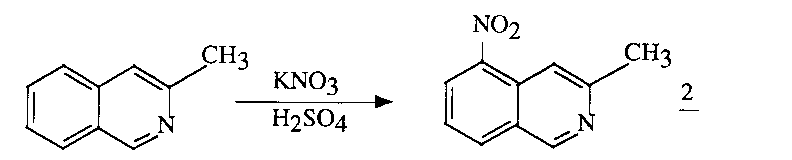

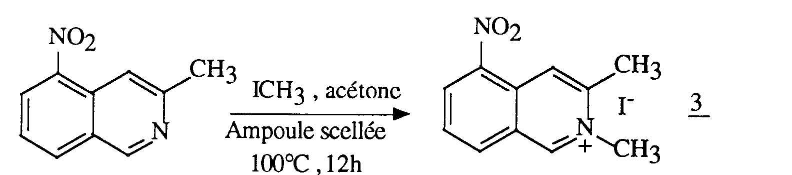

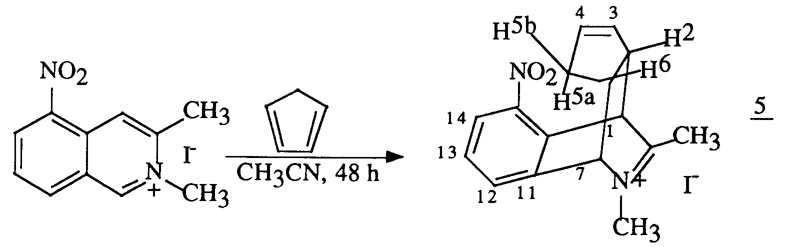

L'iodure de 2,3-diméthyl-5-nitroisoquinolinium 3 (504 mg; 1,53 mmole), dont la synthèse a été décrite dans l'exemple 2, est dissous dans 20 ml d'acétonitrile; on ajoute alors 403 mg (6,1 mmoles) de cyclopentadiène fraîchement distillé et 5 mg d'hydroquinone.

Le mélange réactionnel est laissé 2 jours à température ambiante, puis on recueille l'iodure azatricyclique 5 par précipitation dans l'éther pour obtenir 5 pur.

### 2ème étape :

La réaction de condensation est effectuée dans des conditions identiques aux deux premiers exemples; on obtient alors la spiro-oxazine de formule (Ic) : 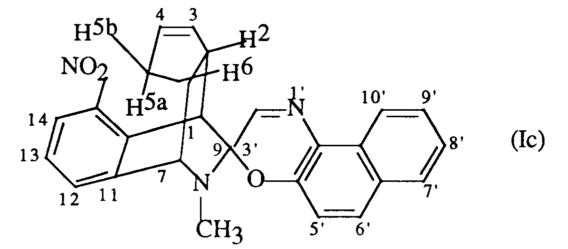
T_{F} = 175-178°C

### Exemple 4

### 1ère étape :

La spiro(azabicyclo-oxazine) correspondante a été préparée selon un mode opératoire similaire à l'exemple 1.

8-éthoxy-9'-méthoxy-2-méthylspiro{syn-5,6-benzo-2-azabicyclo-(2,2,2,)octane-3,3'-[3H]napht-[2,1,b] [1,4]oxazine} :

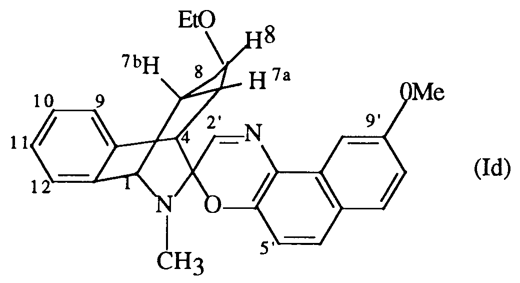

Obtenu à partir de la réaction de condensation de 3 avec le 7-méthoxy- 1-nitroso-2-naphtol.
T_{F} = 152°C

### Exemple 5

La spiro(azabicyclo-oxazine) correspondante a été synthétisée selon un mode préparatoire identique à l'exemple 1, le 1-nitroso-2-naphtol étant remplacé par le 1-nitroso-2-hydroxybenzofurane au cours de la deuxième étape. La couleur de la photomérocyanine résulte d'une bande d'absorption très large dans le visible d'où une teinte se rapprochant de la neutralité. 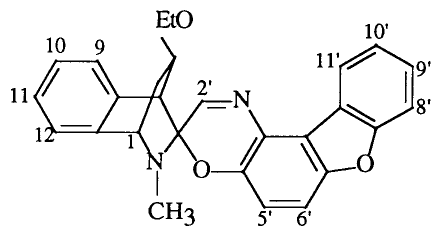
Caractéristiques physiques :
Tr = 169°C
RMN ¹H, 250MHz δ (ppm) (CDCl3, T.M.S.) :
1,04 (t, 3H, OCH₂CH₃); 1,46 (d1, 1H, 7a-H); 2,36 (s, 3H, NMe); 2,91 (ddd, 1H, 7b-H); 3,32-3,57 (m, 2H, OCH₂); 3,81 (dd, 1H, 1-H); 3,89 (d, 1H, 4-H); 4,58 (ddd, 1H, 8-H); 6,98 (s, 1H, 2'-H); 7,08 (d, 1H, 5'-H); 7,24-7,34 (m, 5H); 7,39 (d, 1H, 6'-H); 7,46 (dd, 1H); 8,28 (d, 1H).

### Exemples 6 et 7

La démarche, du point de vue synthèse, est différente. On introduit ici le diénophile sur le motif isoquinolinique (premier étape). Après **cyclisation intramoléculaire** (deuxième étape), on peut alors condenser le cycloadduit obtenu (3 et 4) sur le 1-nitroso-2-naphtol pour obtenir les spiro[azapolycyclane-naphtoxazines] correspondantes (6 et 7).

### 1ère étape :

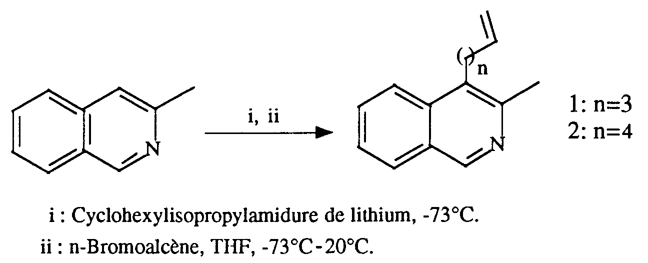

Cette voie réactionnelle a permis la synthèse de la 3-méthyl-4-pent-4-énylisaquinoline 1 et a été appliquée à la 3-méthyl-4-hex-4-énylisoquinoline 2 en vue d'augmenter l'encombrement stérique.

### 2ème étape :

Elle consiste en une thermolyse des 4-énylisoquinolines 1 et 2 :

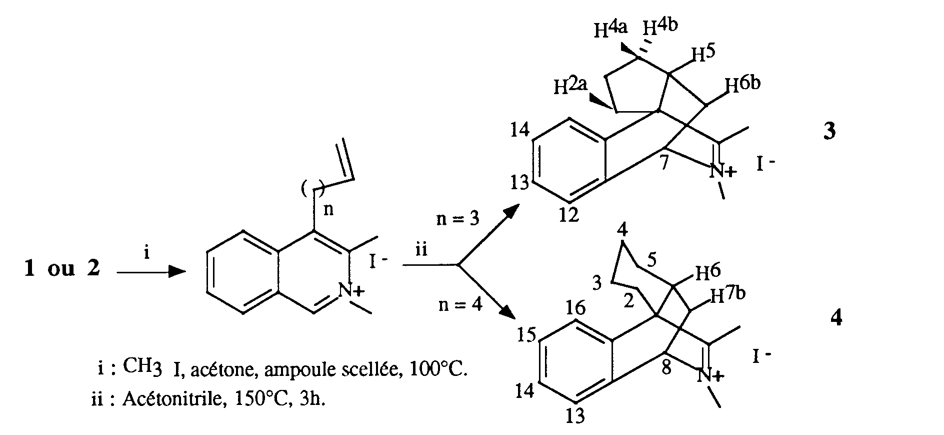

### 3ème étape: Synthèse des spiro[azapolycyclanes-oxazines] :

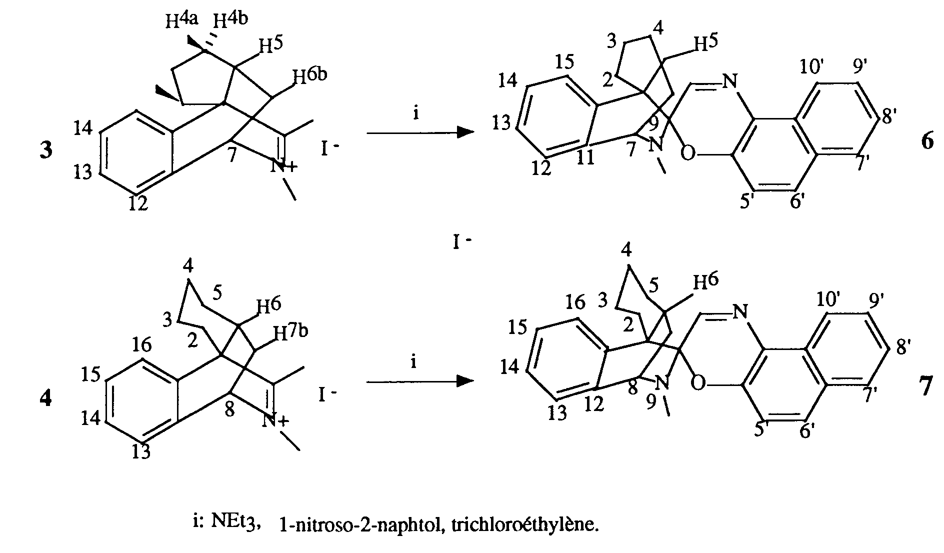

| **PARAMETRES SPECTROCINETIQUES** (mesurés dans le toluène à 25°C) | | | | |
|---|---|---|---|---|
| | Couleur de la photomérocyanine | λₘₐₓ (nm) et λₑ (nm) | Constante cinétique de décoloration thermique K en s⁻¹ | Aₒ (colorabilité à 2,5x10⁻⁵ M) |
| Exemples | | | | |
| 1 | Rosée | (535) 563 | 0,36 | 1,3 |
| 2 | Rosée | 534 (561) | 2,70 | 0,66 |
| 3 | Rosée | 530 - 560 | 1,50 | 0,8 |
| 4 | Rosée | 531 - 558 | 0,37 | 0,9 |
| 5 | Gris-bleutée | (564)-598 (642) | - | - |
| 6 | Rosée | 552-583 | 4,04 | 1,15 |
| 7 | Rosée | 561-590 | 13,08 | 0,89 |

Le spectre d'absorption dans le visible de la forme ouverte de la spirooxazine est caractérisé par :
- le λₘₐₓ (nm) : c'est-à-dire la longueur d'onde au maximum d'absorption.
- le λₑ (nm) indiqué entre parenthèses; il s'agit de la longueur d'onde correspondant à un épaulement du spectre.

## Revendications

1. Composé photochromique, caractérisé par le fait qu'il répond à la formule générale : 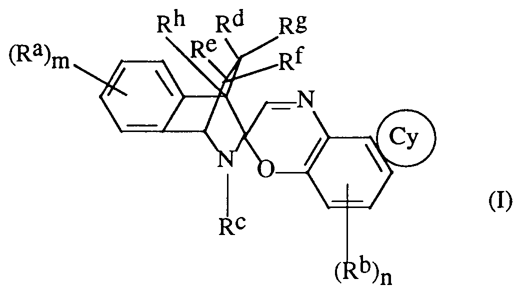 dans laquelle :
R^{a} et R^{b}, indépendamment l'un de l'autre, désignent un atome d'hydrogène; un groupement alkyle; un groupement OR, SR, COR ou COOR dans lesquels R désigne un atome d'hydrogène, un groupement alkyle, aryle ou hétéroaryle;
un groupement amino NR₁R₂ dans lequel R₁ et R₂ désignent, indépendamment l'un de l'autre, un atome d'hydrogène, un groupement alkyle, un groupe cycloalkyle, un groupement aryle, R₁ et R₂ pouvant former avec l'atome d'azote un hétérocycle comportant 4 à 7 chaînons et pouvant contenir en plus un ou plusieurs hétéroatomes choisis parmi soufre, oxygène et azote;
un atome d'halogène, un groupe monohaloalkyle en C₁-C₄; un groupe polyhaloalkyle en C₁-C₄; un groupe NO₂, CN, SCN; SO₃R' où R' désigne hydrogène ou un métal alcalin;
m désigne un entier de 1 à 4 et n vaut 1 ou 2 selon le nombre de substitutions sur le noyau;
Cy désigne un cycle aromatique, de préférence à 5 ou 6 chaînons pouvant être substitué par un ou plusieurs groupements ayant la signification de R^{a} ou un hétérocycle aromatique ou non comportant un ou plusieurs hétéroatomes choisis parmi l'azote, l'oxygène et le soufre ayant 4 à 7 chaînons;
R^{c} désigne un groupe alkyle; un groupe allyle, phényle, arylalkyle mono ou disubstitué par des substituants du type alkyle, alcoxy ou NO₂; un groupe alicyclique éventuellement substitué; un groupe hydrocarboné aliphatique comportant dans sa chaîne un ou plusieurs hétéroatomes tels que oxygène, soufre ou azote;
R^{d}, R^{e}, R^{f}, R^{g} désignent, indépendamment l'un de l'autre, un atome d'hydrogène, un groupement alkyle, un groupe alcoxy ou un groupe thioalkyle; deux de ces radicaux peuvent former un cycloalkyle ou cycloalcényle ayant 4 à 7 chaînons, pouvant comporter un ou plusieurs hétéroatomes choisis parmi l'azote, le soufre, l'oxygène et peuvent être condensés avec un noyau aromatique à 5 ou 6 chaînons pouvant être substitué par un ou plusieurs radicaux ayant la signification de R^{a} et R^{b}.
R^{h} désigne hydrogène ou forme avec R^{d} un cycloalkyle à 5 ou 6 chaînons.

2. Composé selon la revendication 1, caractérisé par le fait que dans la formule (I), les groupements alcoxy et alkyle comportent de préférence 1 à 6 atomes de carbone; les groupements cycloalkyle sont cyclohexyle ou cyclopentyle; le groupement aryle est phényle; le groupement arylalkyle est benzyle; le groupe polyhaloalkyle est CF₃; l'halogène est fluor, brome ou chlore;
les noyaux aromatiques à 5 ou 6 chaînons sont phényle, naphtyle; les noyaux hétérocycles à 4 à 7 chaînons sont les cycles pyridinique, pyrimidique, indolique, thiazolique, imidazolique éventuellement substitués par un groupe alkyle, un groupement phényle ou amino, un groupement carbonyle ou un halogène; le groupement hydrocarboné aliphatique comporte une fonction acide, ester ou alcool.

3. Composé selon la revendication 1 ou 2, caractérisé par le fait que dans la formule (I), les radicaux R^{a} et R^{b} sont choisis parmi l'hydrogène ou NO₂; C_{y} est un cycle aromatique à 6 chaînons éventuellement substitué par un groupe alcoxy; R^{c} est un groupement alkyle; R^{d}, R^{e}, R^{f} et R^{g} désignent un groupement alcoxy ou deux de ces radicaux forment un cycloalcényle.

4. Composé selon la revendication 3, caractérisé par le fait qu'il répond à l'une des formules générales :

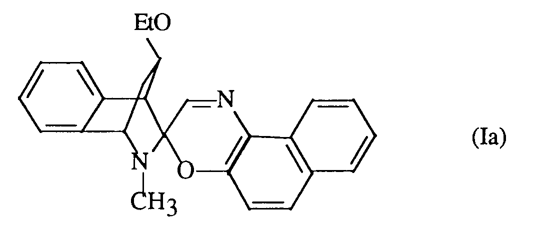

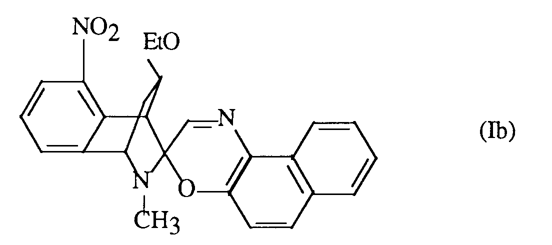

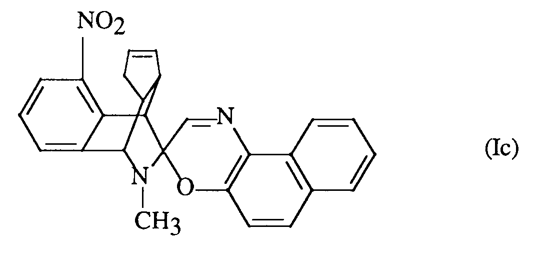

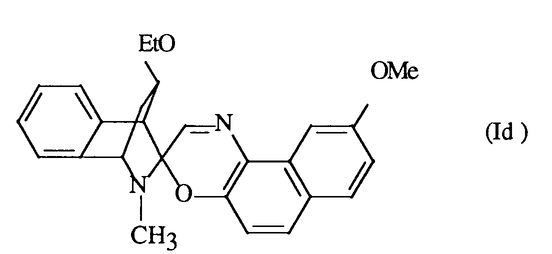

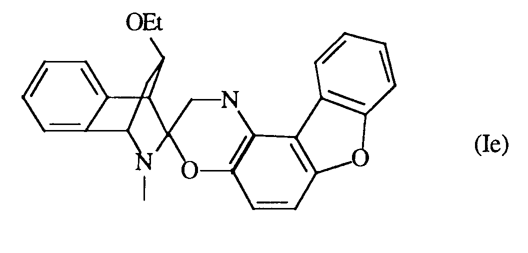

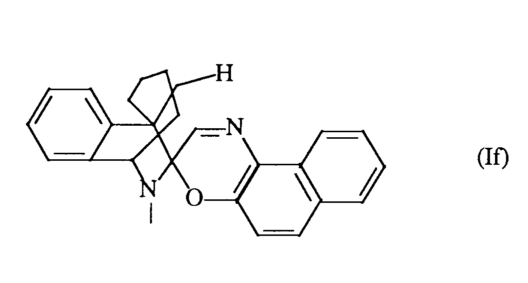

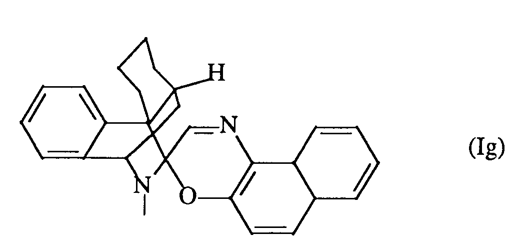

5. Utilisation d'un composé selon l'une quelconque des revendications 1 à 4 comme composé photochromique dans l'optique ophtalmique.

6. Composition destinée à être appliquée sur ou introduite dans un matériau polymère organique transparent, caractérisée par le fait qu'elle contient au moins un composé photochromique tel que défini dans l'une quelconque des revendications 1 à 4, dans des quantités suffisantes pour permettre au matériau exposé à une radiation ultraviolette de changer de couleur.

7. Composition selon la revendication 6, caractérisée par le fait que la composition est sous forme liquide contenant sous forme dissoute ou dispersée, les composés photochromiques selon l'une quelconque des revendications 1 à 3, dans un milieu à base de solvants appropriés pour être appliqués ou introduits dans un matériau polymère transparent.

8. Composition destinée à être appliquée sur ou introduite dans un matériau polymère organique transparent, caractérisée par le fait qu'elle est constituée par une solution incolore ou transparente à base de polymères, de copolymères ou de mélange de polymères transparents dans un solvant organique approprié, contenant au moins un composé photochromique tel que défini dans l'une quelconque des revendications 1 à 4, dans des quantité suffisantes pour permettre au matériau exposé à une radiation ultraviolette de changer de couleur.

9. Matériau solide transparent approprié pour réaliser des lentilles ophtalmiques, caractérisé par le fait qu'il comporte sur la surface et/ou à l'intérieur au moins un composé photochromique tel que défini dans l'une quelconque des revendications 1 à 4, dans des quantités suffisantes pour permettre au matériau exposé à une radiation ultraviolette de changer de couleur.

10. Matériau solide transparent selon la revendication 9, caractérisé par le fait qu'il contient 0,07 à 20% en poids de composés photochromiques.

11. Composition ou matériau solide transparent, selon l'une quelconque des revendications 6 à 10, caractérisée par le fait que le composé photochromique tel que défini dans l'une quelconque des revendications 1 à 4, est utilisé conjointement avec d'autres composés photochromiques donnant lieu à des colorations différentes.

12. Vernis de transfert, caractérisé par le fait qu'il contient au moins un composé tel que défini dans l'une quelconque des revendications 1 à 4.
